# EUROPÄISCHE PATENTSCHRIFT

(11) **EP 1 173 450 B1**
(45) Veröffentlichungstag und Bekanntmachung des Hinweises auf die Patenterteilung: **02.10.2002**
(21) Anmeldenummer: 00936692.3
(22) Anmeldetag: 12.04.2000
(51) Int. Cl.: C07F 9/50, C07C 45/50, B01J 31/28

(54) **VERFAHREN ZUR HERSTELLUNG VON SULFONIERTEN ARYLPHOSPHINEN**
METHOD FOR PRODUCING SULFONATED ARYL PHOSPHINES
PROCEDE DE PREPARATION DE PHOSPHINES D'ARYLE SULFONEES

(30) Priorität: 22.04.1999 DE 19918284; 18.02.2000 DE 10007341
(43) Veröffentlichungstag der Anmeldung: 23.01.2002
(73) Patentinhaber: Celanese Chemicals Europe GmbH, 60439 Frankfurt am Main (DE)
(72) Erfinder: RIEDEL, Michael, 40489 Düsseldorf (DE); HERWIG, Jürgen, 46569 Hünxe (DE); BAHRMANN, Helmut, 46499 Hamminkeln (DE); BERGRATH, Klaus, 46147 Oberhausen (DE); ZGORZELSKI, Wolfgang, 46149 Oberhausen (DE); WIEBUS, Ernst, 46147 Oberhausen (DE); FROHNING, Carl, Dieter, 46485 Wesel (DE); FENSKE, Wilfried, 46499 Hamminkeln (DE); RAMPF, Florian, 84489 Burghausen (DE); ECKL, Robert, 82142 München (DE); BOHNEN, Hans, 47441 Moers (DE); FISCHER, Richard, Louisville,KY (US); SCHALAPSKI, Kurt, 46147 Oberhausen (DE); GREB, Wolfgang, 46535 Dinslaken (DE)
(86) Internationale Anmeldenummer: EP0003241
(87) Internationale Veröffentlichungsnummer: WO00064913

(56) Entgegenhaltungen:
- EP-A- 0 632 047
- WO-A-00/39134
- DE-A- 2 627 354
- HERRMANN W.A.: "New process for the sulfonation of phosphane ligands for catalysts" ANGEWANDTE CHEMIE. INTERNATIONAL EDITION., Bd. 34, Nr. 7, - 13. April 1995 (1995-04-13) Seiten 811-813, XP002147253 VERLAG CHEMIE. WEINHEIM., DE ISSN: 0570-0833

## Beschreibung

Die vorliegende Erfindung betrifft ein Verfahren zur Verringerung des Schwefeldioxid- bzw.Sulfit-Gehaltes in Lösungen sulfonierter Arylphosphine, insbesondere in wäßrigen Lösungen sulfonierter Arylphosphine.

Komplexverbindungen, die als Zentralatom ein Metall der Gruppe VIII des Periodensystems der Elemente und als Liganden Phosphor(III)-Verbindungen wie Phosphine, daneben gegebenenfalls noch weitere zur Komplexbildung befähigte Gruppen enthalten, haben in den letzten Jahren zunehmend als Katalysatoren an Bedeutung gewonnen. So erfolgt die technisch in großem Umfang durchgeführte Umsetzung von Olefinen mit Synthesegas (Mischung aus Wasserstoff und Kohlenmonoxid) zu Aldehyden (Hydroformylierung) in Gegenwart von Katalysatoren, die aus Kobalt oder Rhodium und Triphenylphosphin aufgebaut sind. Entsprechend der Löslichkeit dieser Katalysatoren in organischen Lösungsmitteln verläuft die Umsetzung in homogener Phase.

Anstatt in homogener Phase, kann die Hydroformylierung aber auch in heterogenen Reaktionssystemen durchgeführt werden. Der Einsatz von in Wasser gelösten Katalysatoren hat vielfach den besonderen Vorteil, daß diese Katalysatoren einfach und schonend von den in Wasser wenig oder gar nicht löslichen Reaktionsprodukten abgetrennt und in den Reaktionskessel zurückgeschleust werden können.

Es ist bekannt, Rhodium enthaltende wäßrige Lösungen sulfonierter Arylphosphine als wäßrige Katalysatorlösung für die Herstellung von Aldehyden durch Umsetzung von Olefinen mit Kohlenmonoxid und Wasserstoff (Hydroformylierung) zu verwenden. Ein solches Verfahren ist in der DE-A-26 27 354 beschrieben. Die Rhodium enthaltende wäßrige Lösung enthält als Ligand beispielsweise Tris(m-sulfophenylphosphin) (TPPTS). Der Einsatz von in Wasser gelösten Katalysatoren hat den besonderen Vorteil, dass diese Katalysatoren einfach und schonend durch einfache Phasentrennung von den in Wasser wenig oder gar nicht löslichen Reaktionsprodukten abgetrennt und in den Reaktionskessel zurückgeschleust werden können. Ein derart durchgeführter Hydroformylierungsprozeß wird auch als zweiphasiger Hydroformylierungsprozeß bezeichnet.

Sulfonierte Phenylphosphine werden nach einem Verfahren, das in J. Chem. Soc. (1958), S. 281 u. 282, beschrieben ist, durch die Umsetzung von Triphenylphosphin mit Oleum erhalten. Dabei wird das Reaktionsgemisch zunächst auf einem Wasserbad erwärmt und dann mit Wasser hydrolysiert. Durch die anschließende Neutralisation mit NaOH werden die entsprechenden Natriumsalze des sulfonierten Triphenylphosphins gebildet.

Bei der Umsetzung von Arylphosphinen mit einer Lösung von Schwefeltrioxid in konzentrierter Schwefelsäure, die allgemein als Oleum oder als "rauchende Schwefelsäure" bezeichnet wird, entstehen neben den gewünschten sulfonierten Arylphosphinen auch Sulfitsalze in geringerer Menge. Femer enthält die Reaktionslösung gewisse Mengen an gelöstem Schwefeldioxid. Darüber hinaus reagieren Sulfit oder Schwefeldioxid weiter mit sulfoniertem Arylphosphin zu dem jeweiligen Phosphinsulfid. Unter dem Begriff Sulfit wird im folgenden das Sulfit- bzw. Hydrogensulfitanion der schwefeligen Säure verstanden. Wie bekannt ist, liegt Schwefeldioxid in wäßriger Lösung einmal physikalisch gelöst vor. Zum anderen reagiert Schwefeldioxid mit Wasser unter Bildung von schwefeliger Säure, die unter Protonenabspaltung in das Hydrogensulfit bzw. Sulfitanion übergeht.

Ein gravierender Nachteil des bekannten Verfahrens zur Herstellung sulfonierter Arylphosphine ist die unerwünschte Bildung von Phosphor-Sauerstoff-Verbindungen, die durch Oxidation des dreiwertigen Phosphors zu fünfwertigem Phosphor durch im Oleum gelöstes SO₃ entstehen. Diese Redoxreaktion kann zwar nach der Lehre der EP-A-0 632 047 durch die Zugabe von Borsäure zu dem Reaktionsgemisch verringert werden, jedoch fallen bei dem bekannten Verfahren in erheblichen Mengen Borverbindungen an, die wieder von den Reaktionsprodukten aufwendig abgetrennt werden müssen.

Bei der Redoxreaktion des Schwefeltrioxids mit dem eingesetzten Phosphin entsteht aus dem Schwefeltrioxid als Reduktionsprodukt Schwefeldioxid, und aus dem Phosphin bildet sich das entsprechende Phosphinoxid. Dieses Schwefeldioxid wird bei der, beispielsweise aus EP-A-0 632 047 bekannten Aufarbeitung des Reaktionsgemisches mit einer wäßrigen Natronlauge nicht entfernt. Es verbleibt deshalb als Verunreinigung in Form von Natriumsulfit in der Lösung des sulfonierten Arylphosphins. Man nimmt an, daß dieses Natriumsulfit die Quelle und Ursache für die Verunreinigung von Aldehyden mit Schwefelverbindungen bei der zweiphasigen Hydroformylierung darstellt und daß diese Schwefelverbindungen verantwortlich sind für die Vergiftung von Hydrierkatalysatoren bei der anschließenden Hydrierung der Aldehyde zu Alkoholen .

Man weiß, daß anorganische und organische Schwefelverbindungen (z.B. Butanthiol oder Buttersäurethiobutylester), die sich aus der Reaktion zwischen den während der Hydroformylierungsreaktion gebildeten Aldehyden sowie deren Folgeprodukten, beispielsweise Alkoholen, und Schwefeldioxid bzw. Sulfit bilden, für die Vergiftung von Hydrierkatalysatoren verantwortlich sind, die für die nachfolgende Hydrierung der Aldehyde zu den Oxo-Alkoholen eingesetzt werden. Weiterhin greifen die gebildeten schwefelhaltigen Verbindungen in verschiedene chemische Abläufe nachhaltig und vor allem nachteilig ein, und führen beispielsweise zur Bildung unerwünschter Mischaldole oder Trimerisierungsprodukte aus den Aldehyden.

Es bestand daher die Aufgabe, ein einfaches Verfahren zu entwickeln, mit dem sich sulfonierte Arylphosphine, beispielsweise TPPTS, zuverlässig und frei von Natriumsulfit herstellen lassen.

Gelöst wird diese Aufgabe durch ein Verfahren der eingangs genannten Gattung, dessen kennzeichnendes Merkmal darin zu sehen ist, daß nach der Umsetzung der Arylphosphine mit Oleum das Sulfonierungsgemisch zunächst mit Wasser verdünnt und gekühlt wird, daß dann ein Inertgasstrom so lange durch das verdünnte Sulfonierungsgemisch geleitet wird, bis kein SO₂ mehr aus dem verdünnten Sulfonierungsgemisch mehr freigesetzt wird, und daß dann in üblicher Weise weiter aufgearbeitet wird.

Überraschenderweise hat sich gezeigt, daß durch das erfindungsgemäße Verfahren keine störenden Schwefelverbindungen mehr in den Aldehyden nachweisbar sind, die aus der zweiphasigen Hydroformylierung hervorgehen. Es ist besonders überraschend, daß aus der kleinen Menge an Natriumsulfit die Schwefelverbindungen entstehten, die als Katalysatorgifte fungieren. Sie entstammen also nicht aus den Schwefel enthaltenden Sulfonsäuregruppen der sulfonierten Arylphosphine. Wird die Hydroformylierung mit nach dem erfindungsgemäßen Verfahren sulfonierten Arylphosphinen durchgeführt, gelingt es, die Lebensdauer von Hydrierkatalysatoren, beispielsweise bei der Hydrierung von 2-Ethylhexenal zu 2-Ethylhexanol, um ein Mehrfaches zu verlängern. Durch diese Verlängerung der Lebensdauer der Hydrierkatalysatoren entsteht bei der Herstellung der technischen Alkohole ein erheblicher wirtschaftlicher Vorteil.

Die Sulfonierung wird erfindungsgemäß nach der Lehre der DE-A-26 27 354 durchgeführt. Nach der Verdünnung des Sulfonierungsgemisches aus Arylphosphinen und Oleum mit Wasser bis zu einem Schwefelsäuregehalt von 25 bis 30 % und sofortigen Kühlung wird ein Inertgasstrom durch das verdünnte Sulfonierungsgemisch geleitet. Die Begasung kann dabei chargenweise oder kontinuierlich erfolgen.

Die Menge an Inertgas pro kg an verdünntem Sulfonierungsgemisch und die Zeitdauer der Begasung richten sich nach der Geometrie der Begasungsapparatur. Allgemein gilt, daß die Bedingungen und die Geometrie so zu wählen sind, daß eine weitgehende Befreiung des verdünnten Sulfonierungsgemisches von vorhandenem SO₂ (mindestens 90 %) stattfindet. In einer kontinuierlichen Begasung, beispielsweise in einer gepackten Kolonne, ist eine mittlere Begasungsdauer von 2 bis 100 Minuten, vorzugsweise von 2 bis 20 Minuten, geeignet. Pro kg an verdünntem Sulfonierungsgemisch werden in einer kontinuierlichen Begasungsapparatur eine Gesamtmenge im Bereich von 5 bis 500 I Inertgas, vorzugsweise von 10 bis 200 I, besonders bevorzugt von 20 bis 100 I, benötigt.

Die weitere Aufarbeitung des verdünnten und gereinigten Sulfonierungsgemisches erfolgt in bekannter Weise nach der Lehre der EP-A-0 632 047.

Der Sulfitgehalt in der sulfonierten Arylphosphinlösung liegt dabei bei <0,1 Gew.-%, vorzugsweise bei <0,05 Gew.-%.

So wird das verdünnte Sulfonierungsgemisch mit der Lösung eines wasserunlöslichen Amins, zum Beispiel Triisooctylamin, in einem wasserunlöslichen organischen Lösungsmittel, beispielsweise Toluol, zur Entfernung des Natriumsulfats extrahiert. Anschließend setzt man die erhaltene organische Phase mit der Lösung einer anorganischen Base in Wasser um. Die Base gelangt hierbei in eine der gelösten Aminsalzmenge äquivalenten Menge zum Einsatz. Überschüssige Base ist zu vermeiden, weil sie das Endprodukt verunreinigt. Man erhält auf diese Weise unter Wiedergewinnung des wasserunlöslichen Amins die wäßrige Lösung des sulfonierten Arylphosphins. Das Amin steht für einen erneuten Einsatz wieder zur Verfügung. Die Überführung der sulfonsauren Phosphine in die wäßrige Phase bezeichnet man auch als Re-Extraktion. Vorzugsweise stellt man auf diese Weise eine wäßrige Lösung von Tris(m-sulfophenyl)phosphin Trinatriumsalz (abgekürzt mit TPPTS) her. Nach Rhodiumzugabe zu der wäßrigen TPPTS-Lösung, entweder in Form von metallischem Rhodium oder in Form von Rhodiumverbindungen, wird die wäßrige Lösung als Katalysatorlösung verwendet.

Diese Ausgestaltung des erfindungsgemäßen Verfahrens ist dadurch charakterisiert, dass nur während des Herstellprozesses der sulfonierten Arylphosphine die Möglichkeit besteht, das gebildete Schwefeldioxid zu entfernen. Diese Ausführungsform gestattet es jedoch nicht, aus der fertigen, nach Extraktion mit dem wasserunlöslichen Amin und nach Re-Extraktion mit der anorganischen Base erhaltenen wäßrigen, den wasserlöslichen Liganden enthaltenden Lösung, das Schwefeldioxid zu entfernen. Dieser Nachteil ließe sich nur mit dem Umbau der bestehenden Produktionsanlage zur Herstellung der sulfonierten Arylphosphine überwinden. Der Umbau der bestehenden Produktionsanlage für die Herstellung sulfonierter Arylphosphine ließe sich aber umgehen, wenn der Entgasungsschritt an der die Produktionsanlage verlassenden, fertigen Lösung sulfonierter Arylphosphine ansetzen würde. Femer bietet die bisher beschriebene Ausführungsform des erfindungsgemäßen Verfahrens keine Möglichkeit, Lösungen sulfonierter Arylphosphine, die neben Schwefeldioxid auch Sulfit enthalten, von dem Sulfit zu befreien.

Es bestand daher die weitere Aufgabe ein Verfahren zur Herstellung von Lösungen sulfonierter Arylphosphine bereitzustellen, die einen möglichst geringen Gehalt an Nebenprodukten, insbesondere an Sulfit, Schwefeldioxid und Phosphansulfid aufweisen, und das auch auf fertige, den wasserlöslichen Liganden enthaltende Lösungen anzuwenden ist und sich in einer bereits bestehenden Produktionsanlage für die Herstellung von sulfonierten Arylphosphinen problemlos installieren läßt. Die so erhaltenen, den wasserlöslichen Liganden enthaltenden Lösungen sollen nach Zugabe von Rhodium als Metall oder als Verbindung in dem aus DE-A-26 27 354 bekannten Verfahren zur Herstellung von Aldehyden aus Olefinen in Gegenwart einer wäßrigen Katalysatorlösung nach dem Zweiphasenverfahren direkt einsetzbar sein.

Die Erfindung betrifft daher ebenfalls ein Verfahren zur Herstellung von Lösungen sulfonierter Arylphosphine, erhalten durch Umsetzung der Arylphosphine mit Oleum und Verdünnung des Sulfonierungsgemisches mit Wasser, Extraktion des verdünnten Sulfonierungsgemisches mit der Lösung eines wasserunlöslichen Amins in einem wasserunlöslichen organischen Lösungsmittel, Umsetzung der organischen Phase mit der Lösung einer anorganischen Base in Wasser, das dadurch gekennzeichnet ist, daß die erhaltene wäßrige, sulfonierte Arylphosphine enthaltende Lösung durch Zugabe einer Säure auf einen pH-Wert bis 3 eingestellt wird und daß dann ein Inertgasstrom so lange durch die wäßrige, sulfonierte Arylphosphine enthaltende Lösung geleitet wird, bis kein Schwefeldioxid mehr aus der wäßrigen, sulfonierte Arylphosphine enthaltenden Lösung mehr freigesetzt wird.

Überraschenderweise wurde nun gefunden, dass auch bereits Sulfit enthaltende Lösungen sulfonierter Arylphosphine, die beispielsweise nach dem aus EP-A-0 632 047 bekannten Verfahren zugänglich sind, problemlos wieder von dem Sulfit befreit werden können. Überraschenderweise stellte sich heraus, daß während ihres Herstellverfahrens die sulfonierten Arylphosphine nur langsam mit Sulfit zu den fünfwertigen Phosphor enthaltenden Phosphinsulfiden und Phosphinoxiden abreagieren. Erst nach Zugabe von Rhodium als Metall oder als Verbindung zu der wäßrigen Lösung und Aufpressen von Synthesegas, d.h. einem Gemisch aus Wasserstoff und Kohlenmonoxid, bei erhöhtem Druck und erhöhter Temperatur bilden sich durch Reaktion mit dem anwesenden Sulfit schädliche schwefelhaltige Verbindungen. Die Bildung schädlicher, schwefelhaltiger Verbindungen läßt sich aber unterdrücken, wenn die frisch hergestellte wäßrige Lösung der sulfonierten Arylphosphine nach dem erfindungsgemäßen Verfahren behandelt wird und danach erst Rhodium zugesetzt und Synthesegas bei erhöhter Temperatur aufgepreßt wird. Preßt man zuerst Sythesegas auf, so spricht man von einer Präformierung des Katalysators. Nach dem Präformierungsschritt erfolgt die Olefinzugabe und Reaktion zu den gewünschten Aldehyden. Selbstverständlich ist es auch möglich, die erfindungsgemäß behandelte frische wäßrige Lösung sulfonierter Arylphosphine nach Rhodiumzusatz direkt in den Hydroformylierungsprozeß, der in Gegenwart von Synthesegas und olefinischer Verbindung abläuft, einzusetzen.

Das erfindungsgemäße Verfahren eröffnet die Möglichkeit, frische wäßrige Lösungen sulfonierter Arylphosphine, die bisher noch nicht im Katalyseprozeß verwendet wurden, von dem Sulfit bzw. Schwefeldioxid zu befreien und damit die Bildung schädlicher schwefelhaltiger Verbindungen während des nachgeschalteten Katalyseprozesses zu verhindern.

Für die Durchführung dieses Verfahrens kann man auf die Anlage zurückgreifen, die sich zur kontinuierlichen Inertgasbegasung des verdünnten Sulfonierungsgemisches bewährt hat. Es ist aber auch möglich, die fertige Lösung sulfonierter Arylphosphine in einem Rührkessel chargenweise vorzulegen und nach Säurezugabe Inertgas durch die Lösung zu leiten. Daher bedarf die Implementierung dieses Verfahrens keiner kostspieligen Erweiterung der Produktionsanlage. Die Qualität der Entgasung (Entfernung des Sulfits bzw. Schwefeldioxids) ist vergleichbar mit der Entgasung des verdünnten Sulfonierungsgemisches. Nach Einsatz der erfindungsgemäß behandelten frischen wäßrigen Lösung sulfonierter Arylphosphine in den zweiphasig durchgeführten Hydroformylierungsprozeß lassen sich nur noch geringe Mengen an Schwefelverbindungen in den gewünschten Aldehyden nachweisen. Der Einsatz solcher Aldehyde, die unter Verwendung der erfindungsgemäß behandelten wäßrigen Lösung sulfonierter Arylphosphine in dem zweiphasigen Hydroformylierungsprozeß hergestellt wurden, erweist sich bei der nachfolgenden Hydrierung zu den entsprechenden Alkoholen als besonders vorteilhaft. Der äußerst geringe Gehalt an störenden Schwefelverbindungen in der Hydrierstufe verlängert die Lebensdauer der eingesetzten Hydrierkatalysatoren beträchtlich. So läßt sich beispielsweise die Hydrierung von 2-Ethylhexenal, das nach der Aldolkondensation von n-Butyraldehyd und Wasserabspaltung erhaltene Reaktionsprodukt, zu 2-Ethylhexanol wirtschaftlicher gestalten, da die Lebensdauer der eingesetzten Hydrierkatalysatoren um ein Mehrfaches verlängert wird. Durch diese Verlängerung der Katalysatorstandzeit entsteht bei der Herstellung technisch bedeutender Alkohole ein erheblicher wirtschaftlicher Vorteil aufgrund reduzierter Katalysatorwechselzeiten und damit verbundener geringerer Katalysatoranschaffungs- und entsorgungskosten.

Die Sulfonierung wird nach der Lehre der DE-A-26 27 354 durchgeführt. Man verdünnt das Sulfonierungsgemisch aus Arylphosphinen und Oleum mit Wasser bis zu einem Schwefelsäuregehalt von 25 bis 30 Gew.-%, bezogen auf die Gesamtmasse der Lösung und erhält auf diese Weise das verdünnte Sulfonierungsgemisch. Anschließend wird, wie in EP-A-0 632 047 beschrieben, das verdünnte Sulfonierungsgemisch aufgearbeitet. So wird das verdünnte Sulfonierungsgemisch mit der Lösung eines wasserunlöslichen Amins, zum Beispiel Triisooctylamin, in einem wasserunlöslichen organischen Lösungsmittel, beispielsweise Toluol, zur Entfernung des Natriumsulfats extrahiert. Anschließend setzt man die erhaltene organische Phase mit der Lösung einer anorganischen Base in Wasser um. Die Base gelangt hierbei in eine der gelösten Aminsalzmenge äquivalenten Menge zum Einsatz. Überschüssige Base ist zu vermeiden, weil sie das Endprodukt verunreinigt. Man erhält auf diese Weise unter Wiedergewinnung des wasserunlöslichen Amins die wäßrige Lösung des sulfonierten Arylphosphins. Das Amin steht für einen erneuten Einsatz wieder zur Verfügung. Die Überführung der sulfonsauren Phosphine in die wäßrige Phase bezeichnet man auch als Re-Extraktion. Als Basen für die Überführung der sulfonsauren Phosphine in die Wasserphase sind die Hydroxyde der Alkali- und Erdalkalimetalle, insbesondere Alkalihydroxid, Ammoniak, daneben auch die Alkalicarbonate geeignet.

Auf die aus EP-A-0 632 047 bekannte Arbeitsweise zur Aufarbeitung des verdünnten Sulfonierungsgemisches sei an dieser Stelle ausdrücklich hingewiesen.

Um das Sulfit zu entfernen wird erfindungsgemäß die wäßrige, das sulfonierte Arylphosphin enthaltende Lösung mittels einer Säure auf einen pH-Wert bis 3, vorzugsweise 0 bis 2, eingestellt. Als Säuren lassen sich anorganische oder organische Säuren, beispielsweise Schwefelsäure, Phosphorsäure, Salpetersäure, Salzsäure oder Ameisensäure und Essigsäure in konzentrierter Form oder als Lösung mit einer Konzentration von 1 bis 98 Gew.-% verwenden. Vorzugsweise setzt man eine wäßrige Schwefelsäure mit einer Konzentration von 20 bis 60 Gew.-% ein. Überraschenderweise hat sich gezeigt, daß die Entgasungsraten um so höher sind, je niedriger der pH-Wert eingestellt wurde. Daher ist es für die Durchführung des erfindungsgemäßen Verfahrens besonders vorteilhaft, den pH-Wert auf einen Bereich von 0 bis 2 einzustellen. Die Säurezugabe erfolgt bei einer Temperatur von 0°C bis 70°C, vorzugsweise 10°C bis 40°C. Anschließend wird ein Inertgasstrom durch die Lösung geleitet. Die Begasung kann dabei diskontinuierlich oder kontinuierlich erfolgen.

Nach Abschluß der Begasung wird durch die Zugabe einer wäßrigen alkalisch reagierenden Lösung der pH-Wert auf den Wert einjustiert, den die wäßrige, das sulfonierte Arylphosphin enthaltende Lösung für die anschließende Katalysatorpräformierung oder Hydroformylierungsreaktion aufzuweisen hat. Im allgemeinen liegt der pH-Wert bei der nachfolgenden Hydroformylierungsreaktion in einem Bereich von 5 bis 6,5. Als wäßrige alkalisch reagierende Lösungen verwendet man eine Alkali- oder Erdalkalihydroxid oder Alkali- oder Erdalkâlicarbonatlösung, vorzugsweise eine wäßrige Natronlaugelösung.

Die Begasung der Lösungen wird bei einer Temperatur von 0°C bis 100°C durchgeführt. Besonders bevorzugt ist ein Temperaturbereich von 10°C bis 40°C.

Bei dieser Arbeitsweise werden in der für die Hydroformylierungsreaktion einzusetzenden wäßrigen, sulfonierte Arylphosphine enthaltenden Lösung durch die Säure- und nachträgliche Alkalizugabe zusätzlich Salze gebildet. Diese Salze, zum Beispiel Na₂SO₄, können entweder in der Lösung verbleiben, oder gemäß dem Aufarbeitungsverfahren aus EP-A-0 632 047 durch Extraktion mit der Lösung eines wasserunlöslichen Amins, beispielsweise Triisooctylamin in einem wasserunlöslichen Lösungsmittel und anschließender Re-Extraktion mit der Lösung einer anorganischen Base in Wasser, entfernt werden. Als Basen eignen sich Hydroxyde der Alkali- und Erdalkalimetalle, insbesondere Alkalihydroxid, Ammoniak, daneben auch die Alkalicarbonate.

Die Menge an Inertgas pro kg wäßrige, das sulfonierte Arylphosphin enthaltende Lösung und die Zeitdauer der Begasung richten sich nach der Geometrie der Begasungsapparatur. Allgemein gilt, dass die Bedingungen und die Geometrie so zu wählen sind, dass eine weitgehende Befreiung der wäßrigen, das sulfonierte Arylphosphin enthaltenden Lösung von vorhandenem SO₂ (mindestens 90 %) stattfindet. In einer kontinuierlichen Begasung, beispielsweise in einer gepackten Kolonne, ist eine mittlere Begasungsdauer von 2 bis 100 Minuten, vorzugsweise von 2 bis 20 Minuten, geeignet. Pro kg wäßrige, das sulfonierte Arylphosphin enthaltende Lösung werden in einer kontinuierlichen Begasungsapparatur eine Gesamtmenge im Bereich von 5 bis 500 I Inertgas, vorzugsweise von 10 bis 200 1, besonders bevorzugt von 20 bis 100 I, benötigt. Bei einer diskontinuierlich durchgeführten Begasung werden 5 I bis 1000 I Inertgas pro kg wäßrige Lösung eingesetzt. Dabei beträgt die Inertgasmenge vorzugsweise 10 I bis 200 I und besonders bevorzugt 20 I bis 100 I pro kg wäßrige, das sulfonierte Arylphosphin enthaltende Lösung. Bei der diskontinuierlich durchgeführten Begasung liegt die Begasungsdauer im allgemeinen bei 1 bis 100 Stunden, vorzugsweise 5 bis 50 Stunden und besonders bevorzugt 10 bis 20 Stunden.

Der Sulfitgehalt in der sulfonierten Arylphosphinlösung liegt dabei bei <0,1 Gew.-%, vorzugsweise bei <0,05 Gew.-%

Ausgangsverbindungen für die Sulfonierung nach den verschiedenen Ausgestaltungen der erfindungsgemäßen Verfahren sind Arylphosphine. Unter dieser generellen Bezeichnung sollen Mono-, Di-, Oligo- und Polyphosphine verstanden werden, die mindestens einen aromatischen Rest enthalten. Als aromatische Reste gelten auch solche, die durch einfache C-C-Verbindungen miteinander verknüpft sind wie Biphenyl, aber insbesondere auch annellierte Ringsysteme wie Naphthyl- oder Indenylreste.

Die aromatischen Reste können zusätzlich einen oder mehrere Substituenten tragen wie Chlor-, Fluor-, Alkyl- mit 1 bis 10 C-Atomen, Alkoxy- mit 1 bis 10 C-Atomen und Nitrogruppen. Beispiele für Monophosphine, die nach dem erfindungsgemäßen Verfahren sulfoniert werden können, sind Dimethylphenyl-, Methyldiphenyl- und Triphenylphosphin. Die Gruppe der Diphosphine wird beispielhaft durch 2,2'-Bis(diphenlyphosphinomethyl)-biphenyl oder durch 2,2'-Bis(diphenlyphosphinomethyl)-binaphthyl beschrieben. Unter den erfindungsgemäß geeigneten Arylphosphinen werden darüber hinaus auch Verbindungen des dreiwertigen Phosphors verstanden, in denen das Phosphoratom Bestandteil eines Ringsystems ist. Für diese Verbindungsklassen stehen als Beispiele mit aromatischen Resten substituiertes Phosphorbenzol und aryl- und/oder alkylsubstituierte Phosphole. Beispiele für derartige Verbindungen sind in J. Mol. Cat. A, 116 (1997), S. 3 - 26, beschrieben.

Als Inertgase für die Begasung des verdünnten Sulfonierungsgemisches oder der angesäuerten wäßrigen, die sulfonierte Arylphosphine enthaltende Lösung können alle Gase verwendet werden, die chemisch nicht mit der zugesetzten Säure oder den gelösten Arylphosphinen reagieren. Beispiele für Inertgase sind Stickstoff, die Edelgase Helium, Neon, Argon, Xenon und Krypton, Kohlendioxid, Kohlenmonoxid oder Wasserstoff, wobei Stickstoff und die preisgünstigeren Edelgase bevorzugt sind.

Die verschiedenen Ausgestaltungen der erfindungsgemäßen Verfahren eignet sich in besonderem Maße zur Behandlung von wäßrigen TPPTS-Lösungen.

Der mit SO₂ beladene Inertgasstrom kann nicht unbehandelt in die Atmosphäre abgeleitet werden. Er wird deshalb durch eine Waschlösung geleitet, in der das SO₂ absorbiert wird. Diese Waschlösung kann eine basische Flüssigkeit sein, beispielsweise eine Natriumkarbonatlösung oder eine NaOH-Lösung. Man kann aber auch eine Abfallschwefelsäure als Waschflüssigkeit verwenden.

Die Erfindung wird anhand der nachfolgend angegebenen Ausführungsbeispiele für die Fachwelt noch deutlicher erläutert, ohne daraus Beschränkungen auf die konkret dargestellten Ausführungsformen abzuleiten.

### Beispiel 1

Entgasung von verdünntem Sulfonierungsgemisch aus der Sulfonierung von Triphenylphosphin (TPPTS-Gehalt circa 3 %, Schwefelsäure circa 25 %).

962,3 g des obigen verdünnten Sulfonierungsgemisches wurden in eine 106 cm lange Blasensäule mit einem Innendurchmesser von 4 cm gefüllt und bei Raumtemperatur mit Stickstoff bei einer Strömungsgeschwindigkeit von 20 l/h behandelt.

Der aus der Blasensäule austretende Gasstrom wurde durch eine Waschflasche geleitet, die mit 200 g einer 2,7 %-igen Natriumkarbonatlösung befüllt war. Aus dieser Waschflasche wurden Proben gezogen und mit Hilfe der lonenchromatographie auf Sulfit und Sulfat untersucht. Es zeigte sich, daß alles Sulfat in der Waschflasche aus der Oxidation von Sulfit stammt, da auch nach sehr langer Begasungszeit die Menge an Sulfat nicht weiter zunimmt. Dies müßte aber geschehen, wenn Schwefelsäure als Aerosol mit dem Inertgas aus dem verdünnten Sulfonierungsgemisch ausgetragen würde. Die Messergebnisse im einzelnen sind in der nachfolgenden Tabelle dargestellt.

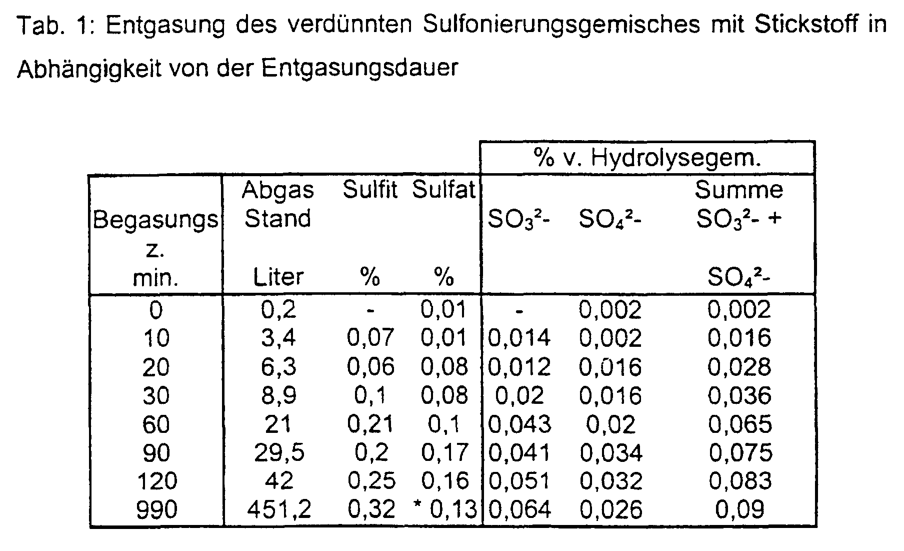

Nach Aufarbeitung gemäß der Lehre der EP-A-0 632 047 wurde eine 15 %ige TPPTS-Lösung erhalten. Der Sulfitgehalt dieser Lösung lag unterhalb der Nachweisgrenze von 0,01 Gew.-%, gemessen nach der Methode der lonenchromatographie.

### Beispiel 2 (Vergleichsbeispiel)

Beispiel 1 wurde nachgestellt, jedoch mit dem Unterschied, daß eine Begasung des verdünnten Sulfonierungsgemisches nicht durchgeführt wurde. Nach Aufarbeitung gemäß EP-A-0 632 047 wurde eine 15 %-ige TPPTS-Lösung erhalten, an der ein Sulfitgehalt von 0,20 Gew.-% gemessen wurde.

### Beispiel 3

Die TPPTS-Lösung aus Beispiel 1 wurde in einer kontinuierlichen Hydroformylierung mit Propen als Olefin, 50 bar Synthesegas, 300 ppm Rhodium, TPPTS 300 mmol/kg eingesetzt. Bei einer Reaktionstemperatur von 134°C wurde im gesammelten Butanal nach einer Reaktionszeit von 211 h ein Schwefelwert von 1,7 ppm festgestellt

### Beispiel 4 (Vergleichsbeispiel)

Die TPPTS-Lösung aus Beispiel 2 wurde in einer kontinuierlichen Hydroformylierung nach Beispiel 3 eingesetzt. Nach 230 h Reaktionszeit wurde in dem entstandenen Butanal ein Schwefelwert von 6,5 ppm festgestellt.

### Beispiel 5

Kontinuierliche Entgasung siehe Abbildung 1.

In einer Desorberkolonne A von 8 m Länge und 10 cm Innendurchmesser mit einer Füllung aus 15 mm Pall Ringen werden am Kopf 200 l/h des Hydrolysegemisches mit vergleichbarer Zusammensetzung wie Beispiel 1 gegeben. Im Gegenstrom werden von unten 8,8 m³/h Stickstoff in die Kolonne eingebracht. Nach Einstellung des Gleichgewichtes wurde eine Entgasung von 92 % des ursprünglich vorhandenen Schwefeldioxids festgestellt. Nach Aufarbeitung gemäß EP-A-0 632 047 wurde eine 15 %-ige TPPTS-Lösung erhalten, an der ein Sulfitgehalt von 0,02 Gew.-% gemessen wurde.

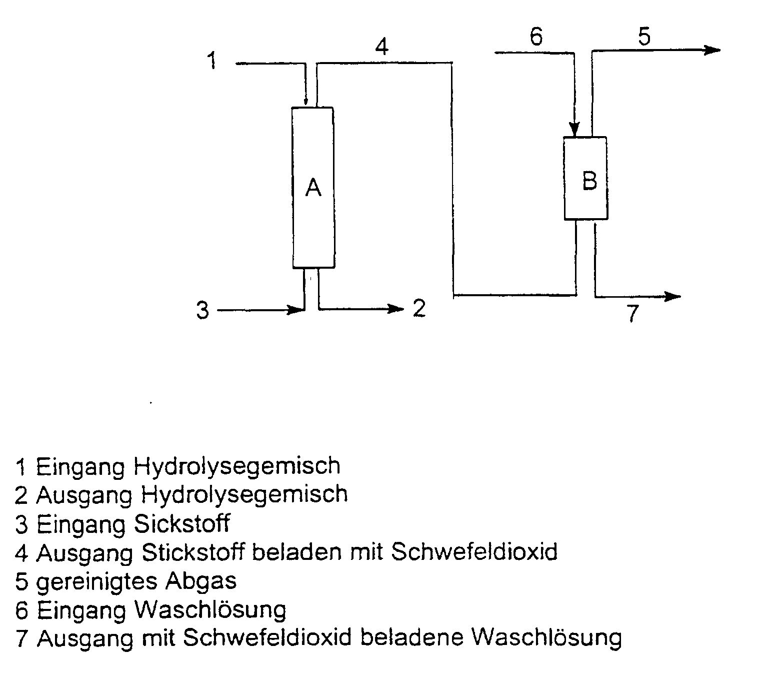

### Beispiel 6

Entgasung einer TPPTS-Lösung (Lösung von Tris(m-sulfophenyl)phosphin Trinatriumsalz) bei einem pH-Wert von 0 (TPPTS-Gehalt circa 27 Gew.-%, Sulfitgehalt 0,338 Gew.-%).

2500 g einer TPPTS-Lösung, sulfoniert nach dem aus DE-A-26 27 454 und aufgearbeitet nach dem aus EP-A-0 632 047 bekannten Verfahren, enthaltend 8,46 g SO₃²⁻ wurden mit 314g H₂SO₄ (65%ig) versetzt und in einem 4 l Dreihalskolben mit Frittenaufsatz und Frittenfilter intensiv mit Stickstoff begast. Die Begasung wurde bei 20°C mit Stickstoff bei einer Strömungsgeschwindigkeit von 20 l/h durchgeführt.

Der austretende Gasstrom wurde durch eine Waschflasche geleitet, die mit 400 g einer Natriumkarbonatlösung/Natriumhydrogencarbonatlösung (0,4 mol Na₂CO₃, 0,15 mol NaHCO₃) befüllt war. Aus dieser Waschflasche wurden Proben gezogen und mit Hilfe der lonenchromatographie auf Sulfit und Sulfat untersucht, wobei durch Oxidation alles Sulfit in das Sulfat überführt wurde. Es zeigte sich, dass der gesamte Sulfatgehalt in der Natriumkarbonatlösung/Natriumhydrogencarbonatlösung aus der Oxidation von Sulfit stammt, da auch nach sehr langer Begasungszeit die Menge an Sulfat nicht weiter zunahm. Dies hätte aber geschehen müssen, wenn Schwefelsäure als Aerosol mit dem Inertgas aus der angesäuerten wäßrigen TPPTS-Lösung ausgetragen worden wäre. Die Messergebnisse im einzelnen sind in der nachfolgenden Tabelle dargestellt. Die Meßwerte in der vorletzten Spalte stellen die auf Sulfit umgerechneten Sulfat-Werte dar, die gemäß Analyse in der Natriumkarbonatlösung/Natriumhydrogencarbonatlösung (drittletzte Spalte) gefunden wurden.

**Tab. 2:**

| Entgasung einer wäßrigen TPPTS-Lösung mit Stickstoff in Abhängigkeit von der Entgasungsdauer | | | | | |
|---|---|---|---|---|---|
| Laufzeit | Eings. TPPTS-Lsg. | Na₂CO₃/ NaHCO₃ Lsg. | SO₄²⁻ in der Na₂CO₃/ NaHCO₃ Lsg. | SO₃²⁻ Austrag aus TPPTS-Lsg. | Entgasungsrate |
| [h] | [g] | [g] | [Gew.-%] | [g] | [%] |
| 0 | 2814 | 400,4 | < 0,01 | <0,03 | 0 |
| 1 | 2809 | 399,2 | 1,24 | 4,13 | 48,8 |
| 3 | 2804 | 398,0 | 2,05 | 6,82 | 80,6 |
| 24 | 2799 | 397,8 | 2,4 | 7,96 | 194,1 |

(Entgasungsrate bedeutet: SO₃²⁻-Austrag in Gramm aus der TPTS-Lösung, bezogen auf die SO₃²⁻-Menge in Gramm in der TPPTS-Lösung vor der Entgasung).

2742 g der begasten TPPTS-Lösung wurden mit 5928 g einer Mischung aus Triisooctylamin/Toluol mit einem Triisooctylamin-Gehalt von 28 Gew.-% in einem 10I Dreihalskolben 1 Stunde bei 32°C gerührt. Nach Abtrennung der organischen Phase wurde diese mit einer wäßrigen 10%-igen Natronlaugelösung re-extrahiert. Die anschließende Re-Extration diente zur Abtrennung des durch das Ansäuern mit Schwefelsäure eingetragenen Sulfats. Die drei Reextraktionsschritte sind in der nachfolgenden Tabelle aufgeführt.

**Tabelle 3:**

| Reextraktion der organischen Phase, erhalten aus Extraktion der angesäuerten und begasten, wäßrigen TPPTS-Lösung. | | | |
|---|---|---|---|
| | 1. Stufe | 2. Stufe | 3. Stufe |
| Einstellung bis pH = | 3,54 | 8,00 | 12,90 |
| Verbrauch NaOH [g] | 405 | 1701 | 160 |
| Auswaage TPPTS-Lsg [g] | 166,0 | 2574,8 | 166,0 |
| P³-Gehalt [mmol] | 0 | 1296,3 | 3,2 |
| Ausbeute [%] | 0 | 97,2 | 0,2 |

Wie die Tabelle 3 zeigt, geht in einem pH-Bereich bis 3,54 Einheiten das Sulfat aus der organischen in die wäßrige Phase über, während bei der Re-Extraktion im pH-Bereich von 3,54 bis 8,00 das gewünschte TPPTS aus der organischen in die wäßrige Phase übergeht.

### Beispiel 7

Entgasung einer TPPTS-Lösung bei einem pH-Wert von 1 (TPPTS-Gehalt circa 27 Gew.-%, Sulfitgehalt 0,34 Gew.-%).

2500 g einer, wie im Beispiel 6 eingesetzten TPPTS-Lösung und enthaltend 8,5g SO₃²⁻ wurden mit 18g H₂SO₄ (65%ig) versetzt und in einem 4 I Dreihalskolben mit Frittenaufsatz und Frittenfilter intensiv mit Stickstoff begast. Die Begasung wurde bei 20°C mit Stickstoff bei einer Strömungsgeschwindigkeit von 20 l/h durchgeführt.

Der aus dem Dreihalskolben austretende Gasstrom wurde durch eine Waschflasche geleitet, die mit 400 g einer Natriumkarbonatlösung/Natriumhydrogencarbonatlösung (0,4 mol Na₂CO₃, 0,15 mol NaHCO₃) befüllt war. Aus dieser Waschflasche wurden Proben gezogen und mit Hilfe der lonenchromatographie auf Sulfit und Sulfat untersucht, wobei durch Oxidation alles Sulfit in das Sulfat überführt wurde. Es zeigte sich, dass der gesamte Sulfatgehalt in der Natriumkarbonatlösung/Natriumhydrogencarbonatlösung aus der Oxidation von Sulfit stammt, da auch nach sehr langer Begasungszeit die Menge an Sulfat nicht weiter zunahm. Dies hätte aber geschehen müssen, wenn Schwefelsäure als Aerosol mit dem Inertgasstrom aus der angesäuerten, wäßrigen TPPTS-Lösung mitgerissen worden wäre. Die Messergebnisse im einzelnen sind in der nachfolgenden Tabelle dargestellt.

**Tabelle 4:**

| Entgasung einer auf pH 1 eingestellten wäßrigen TPPTS-Lösung in Abhängigkeit von der Entgasungsdauer | | | | | |
|---|---|---|---|---|---|
| Laufzeit | Eings. TPPTS-Lsg. | Na₂CO₃/ NaHCO₃ Lsg. | SO₄²⁻ in der Na₂CO₃/ NaHCO₃ Lsg. | SO₃²⁻ Austrag aus TPPTS-Lsg. | Entgasungsrate |
| [h] | [g] | [g] | [%] | [g] | [%] |
| 0 | 2518 | 399 | <0,01 | <0,03 | 0 |
| 1 | 2516 | 397,8 | 0,16 | 0,53 | 6,3 |
| 3 | 2511 | 396,6 | 1,39 | 4,61 | 54,2 |
| 24 | 12509 | 387,8 | 2,3 | 7,48 | 88,1 |

Die begaste TPPTS-Lösung wurde nicht wie im Beispiel 6 mittels Extraktion und Reextraktion aufgearbeitet, sondern durch Zugabe einer 10%igen NaOH-Lösung auf pH 6,5 eingestellt. Das dabei gebildete Na₂SO₄ wurde in der Lösung belassen.

### Beispiel 8

Entgasung einer TPPTS-Lösung bei einem pH-Wert von 2 (TPPTS-Gehalt circa 27 Gew.-%, Sulfitgehalt 0,34 Gew.-%).

2500 g einer, wie im Beispiel 6 eingesetzten TPPTS-Lösung, enthaltend 8,5g SO₃²⁻ wurden mit 4,72g H₂SO₄ (65%ig) versetzt und in einem 4 I Dreihalskolben mit Frittenaufsatz und Frittenfilter intensiv mit Stickstoff begast. Die Begasung wurde bei 20°C mit Stickstoff bei einer Strömungsgeschwindigkeit von 20 l/h durchgeführt.

Der aus dem Dreihalskolben austretende Gasstrom wurde durch eine Waschflasche geleitet, die mit 400 g einer Natriumkarbonatlösung/Natriumhydrogencarbonatlösung (0,4 mol Na₂CO₃, 0,15 mol NaHCO₃) befüllt war. Aus dieser Waschflasche wurden Proben gezogen und mit Hilfe der lonenchromatographie auf Sulfit und Sulfat untersucht, wobei durch Oxidation alles Sulfit in das Sulfat durch Oxidation überführt wurde. Es zeigte sich, dass der gesamte Sulfatgehalt in der Natriumkarbonatlösung/Natriumhydrogencarbonatlösung aus der Oxidation von Sulfit stammt, da auch nach sehr langer Begasungszeit die Menge an Sulfat nicht weiter zunahm. Dies hätte aber geschehen, wenn Schwefelsäure als Aerosol mit dem Inertgasstrom aus der angesäuerten, wäßrigen TPPTS-Lösung mitgerissen worden wäre. Die Messergebnisse im einzelnen sind in der nachfolgenden Tabelle dargestellt.

**Tab. 5:**

| Entgasung einer auf pH 2 eingestellten TPPTS-Lösung in Abhängigkeit von der Entgasungsdauer. | | | | | |
|---|---|---|---|---|---|
| Laufzeit | Eings. TPPTS-Lsg. | Na₂CO₃/ NaHCO₃ Lsg. | SO₄²⁻ in der Na₂CO₃/ NaHCO₃ Lsg. | SO₃²⁻ Austrag aus TPPTS-Lsg. | Entgasungsrate |
| [h] | [g] | [g] | [%] | [g] | [%] |
| 0 | 2500 | 402,9 | < 0,01 | <0,03 | 0 |
| 1 | 2498 | 401,8 | 0,12 | 0,40 | 4,8 |
| 3 | 2494 | 399,8 | 0,47 | 1,58 | 18,8 |
| 24 | 2491 | 388,7 | 1,76 | 5,85 | 69,6 |

Die begaste TPPTS-Lösung wurde nicht wie im Beispiel 6 mittels Extraktion und Reextraktion aufgearbeitet, sondern mittels einer 10%igen NaOH-Lösung auf pH 6,5 eingestellt. Das dabei gebildete Na₂SO₄ wurde in der Lösung belassen.

Wie aus den Tabellen 2, 4 und 5 ersichtlich, beobachtet man mit sinkendem pH-Wert eine steigende Entgasungsrate.

### Beispiel 9

Hydroformylierung mit der gemäß Beispiel 6 behandelten TPPTS-Lösung

Eine gemäß Beispiel 6 behandelte TPPTS-Lösung (Re-extraktion nach der Entgasungssstufe) mit einem TPPTS-Gehalt von 300 mmol/kg wurde in einer kontinuierlichen Hydroformylierung mit Propen als Olefin, bei 50 bar Synthesegasdruck verwendet. Die Rhodiumkonzentration in der wäßrigen Katalysatorlösung betrug 300 ppm Rhodium. Bei einer Reaktionstemperatur von 134°C wurde im angefallenen Butanal nach einer Reaktionszeit von 211 h ein Schwefelgehalt von 1,6 ppm festgestellt.

### Beispiel 10

Hydroformylierung mit der gemäß Beispiel 8 behandelten TPPTS-Lösung.

Eine gemäß Beispiel 8 behandelte TPPTS-Lösung (keine Re-extraktion nach der Entgasungssstufe) mit einem TPPTS-Gehalt von 300 mmol/kg wurde in einer kontinuierlichen Hydroformylierung mit Propen als Olefin, bei 50 bar Synthesegasdruck verwendet. Die Rhodiumkonzentration in der wäßrigen Katalysatorlösung betrug 300 ppm Rhodium. Bei einer Reaktionstemperatur von 134°C wurde im angefallenen Butanal nach einer Reaktionszeit von 211 h ein Schwefelgehalt von 2,5 ppm festgestellt

### Beispiel 11

Vergleichsbeispiel mit einer nicht entgasten TPPTS-Lösung.

Die unbehandelte TPPTS-Lösung aus Beispiel 6 mit einem Sulfit-Gehalt von 0,38 Gew.-% und einem TPPTS-Gehalt von 300 mmol/kg wurde ohne Durchführung der erfinderischen Ansäuerung und Begasung in einer kontinuierlichen Hydroformylierung mit Propen als Olefin bei 50 bar Synthesegasdruck verwendet. Der Rhodiumgehalt betrug 300 ppm, bezogen auf die wäßrige Lösung. Nach 230 h Reaktionszeit wurde in dem angefallenen Butanal ein Schwefelgehalt von 9,8 ppm festgestellt.

Wie ein Vergleich der Beispiele 9 und 10 mit dem Vergleichsbeispiel 11 belegt, liefert die Verwendung einer erfindungsgemäß behandelten wäßrigen TPPTS-Lösung in der Hydroformylierungsreaktion Aldehyde, die einen deutlich erniedrigten Schwefelgehalt aufweisen. Der Einsatz solcher Aldehyde bei der Alkoholherstellung in nachgeschalteten Hydrierprozessen ist besonders vorteilhaft, da die Vergiftung der Hydrierkatalysatoren mit Schwefelverbindungen vermindert wird. Ferner zeigen Aldehyde mit einem geringen Schwefelgehalt eine höhere Stabilität gegenüber Trimerisierungs- und Kondensationsreaktionen bei der Lagerung.

## Patentansprüche

1. Verfahren zur Herstellung von sulfonierten Arylphosphinen durch Umsetzung von Arylphosphinen mit Oleum, **dadurch gekennzeichnet, daß** nach der Umsetzung der Arylphosphine mit Oleum das Sulfonierungsgemisch zunächst mit Wasser verdünnt und gekühlt wird, daß dann ein Inertgasstrom so lange durch das verdünnte Sulfonierungsgemisch geleitet wird, bis kein SO₂ mehr aus dem verdünnten Sulfonierungsgemisch mehr freigesetzt wird, und daß dann in üblicher Weise weiter aufgearbeitet wird.

2. Verfahren nach Anspruch 1, **dadurch gekennzeichnet, daß** als Ausgangsverbindungen für die Sulfonierung Arylphosphine wie Mono-, Di-, Oligo- und Polyphosphine eingesetzt werden, die mindestens einen aromatischen Rest enthalten.

3. Verfahren nach Anspruch 1 oder 2, **dadurch gekennzeichnet, daß** die Verdünnung des Sulfonierungsgemisches aus Arylphosphinen und Oleum mit Wasser bis zu einem Schwefelsäuregehalt von 25 bis 30 % erfolgt.

4. Verfahren nach einem der Ansprüche 1 bis 3, **dadurch gekennzeichnet, daß** das verdünnte Sulfonierungsgemisch sofort gekühlt wird und daß ein kontinuierlicher Inertgasstrom durch das verdünnte Sulfonierungsgemisch geleitet wird.

5. Verfahren nach einem der Ansprüche 1 bis 4, **dadurch gekennzeichnet, daß** als Inertgase Stickstoff, die Edelgase Helium, Neon, Argon, Xenon und Krypton, Kohlendioxid, Kohlenmonoxid oder Wasserstoff verwendet werden, vorzugsweise Stickstoff oder Edelgase.

6. Verfahren nach einem der Ansprüche 1 bis 5, **dadurch gekennzeichnet, daß** die Menge an Inertgas pro kg an verdünntem Sulfonierungsgemisch im Bereich von 5 bis 500 I Inertgas, vorzugsweise von 10 bis 200 I, besonders bevorzugt von 20 bis 100 I, eingestellt wird.

7. Verfahren nach einem der Ansprüche 1 bis 6, **dadurch gekennzeichnet, daß** die Zeitdauer der Begasung im Bereich von 2 bis 100 Minuten, vorzugsweise von 2 bis 20 Minuten, eingestellt wird und wobei nach üblicher Aufarbeitung in der sulfonierten Arylphosphinlösung der Sulfitgehalt bei <0,1 Gew.-%, vorzugsweise <0,05 Gew.-%, liegt.

8. Verfahren nach einem der Ansprüche 1 bis 7, **dadurch gekennzeichnet, daß** der mit SO₂ beladene Inertgasstrom durch eine Waschlösung geleitet wird, in der das SO₂ absorbiert wird, wobei die Waschlösung vorzugsweise eine basische Flüssigkeit ist wie Natriumkarbonatlösung oder eine NaOH-Lösung oder eine Abfallschwefelsäure.

9. Verfahren zur Herstellung von Lösungen sulfonierter Arylphosphine, erhalten durch Umsetzung der Arylphosphine mit Oleum und Verdünnung des Sulfonierungsgemisches mit Wasser, Extraktion des verdünnten Sulfonierungsgemisches mit der Lösung eines wasserunlöslichen Amins in einem wasserunlöslichen organischen Lösungsmittel, Umsetzung der organischen Phase mit der Lösung einer anorganischen Base in Wasser, **dadurch gekennzeichnet, daß** die erhaltene wäßrige, sulfonierte Arylphosphine enthaltende Lösung durch Zugabe einer Säure auf einen pH-Wert bis 3 eingestellt wird und daß dann ein Inertgasstrom so lange durch die wäßrige, sulfonierte Arylphosphine enthaltende Lösung geleitet wird, bis kein Schwefeldioxid mehr aus der wäßrigen, sulfonierte Arylphosphine enthaltenden Lösung mehr freigesetzt wird.

10. Verfahren nach Anspruch 9, **dadurch gekennzeichnet, daß** als Arylphosphine als Ausgangsverbindungen für die Sulfonierung Mono-, Di-, Oligo- und Polyphosphine eingesetzt werden, die mindestens einen aromatischen Rest enthalten.

11. Verfahren nach Anspruch 9 oder 10, **dadurch gekennzeichnet, daß** als Monophosphine Dimethylphenylphosphin, Methyldiphenylphosphin und Triphenylphosphin verwendet werden.

12. Verfahren nach einem der Ansprüche 9 bis 11, **dadurch gekennzeichnet, daß** die wäßrige, sulfonierte Arylphosphine enthaltende Lösung durch Zugabe einer Säure auf einen pH-Wert von 0-2 eingestellt wird.

13. Verfahren nach einem der Ansprüche 9 bis 12, **dadurch gekennzeichnet, daß** man die wäßrige, sulfonierte Arylphosphine enthaltende Lösung mit anorganischen oder organischen Säuren versetzt.

14. Verfahren nach einem der Ansprüche 9 bis 13, **dadurch gekennzeichnet, daß** man als Säure Schwefelsäure, Phosphorsäure, Salpetersäure, Salzsäure, Ameisensäure oder Essigsäure verwendet.

15. Verfahren nach einem der Ansprüche 9 bis 14, **dadurch gekennzeichnet, daß** durch die wäßrige, sulfonierte Arylphosphine enthaltende Lösung ein Inertgasstrom kontinuierlich oder diskontinuierlich geleitet wird.

16. Verfahren nach einem der Ansprüche 9 bis 15, **dadurch gekennzeichnet, daß** als Inertgase Stickstoff, die Edelgase Helium, Neon, Argon, Xenon und Krypton, Kohlendioxid, Kohlenmonoxid oder Wasserstoff verwendet werden, vorzugsweise Stickstoff oder Edelgase.

17. Verfahren nach einem der Ansprüche 9 bis 16, **dadurch gekennzeichnet, daß** bei der kontinuierlich durchgeführten Begasung die Zeitdauer der Begasung im Bereich von 2 bis 100 Minuten, vorzugsweise von 2 bis 20 Minuten, eingestellt wird und die Menge an Inertgas pro kg wäßrige, das sulfonierte Arylphosphin enthaltende Lösung im Bereich von 5 bis 500 I Inertgas, vorzugsweise von 10 bis 200 I, besonders bevorzugt von 20 bis 100 I Inertgas eingestellt wird wobei in der wäßrigen, sulfonierte Arylphosphine enthaltenden Lösung der Sulfitgehalt bei <0,1 Gew.-%, vorzugsweise <0,05 Gew.-%, liegt.

18. Verfahren nach einem der Ansprüche 9 bis 17, **dadurch gekennzeichnet, daß** bei der diskontinuierlich durchgeführten Begasung die Zeitdauer der Begasung im Bereich von 1 bis 100 Stunden, vorzugsweise von 5 bis 50 Stunden und besonders bevorzugt von 10 bis 20 Stunden, eingestellt wird und die Menge an Inertgas pro kg wäßrige, das sulfonierte Arylphosphin enthaltende Lösung im Bereich von 5 bis 1000 I Inertgas, vorzugsweise von 10 bis 200 I, besonders bevorzugt von 20 bis 100 I Inertgas eingestellt wird, wobei in der wäßrigen, sulfonierte Arylphosphine enthaltenden Lösung der Sulfitgehalt bei <0,1 Gew.-%, vorzugsweise <0,05 Gew.-%, liegt.

19. Verfahren nach einem der Ansprüche 9 bis 18, **dadurch gekennzeichnet, daß** der mit Schwefeldioxid beladene Inertgasstrom durch eine Waschlösung geleitet wird, in der Schwefeldioxid absorbiert wird, wobei die Waschlösung vorzugsweise eine basische Flüssigkeit ist wie Natriumkarbonatlösung oder eine Natriumhydroxidlösung oder eine Abfallschwefelsäure.

20. Wäßrige Katalysatorlösung enthaltend Rhodium und eine wäßrige, sulfonierte Arylphosphine enthaltende Lösung, erhältlich nach einem der Ansprüche 9 bis 19, zur Hydroformylierung von olefinisch ungesättigten Verbindungen.

21. Verwendung der wäßrigen Katalysatorlösung nach Anspruch 20 zur Hydroformylierung von olefinisch ungesättigten Verbindungen.

## Claims

1. A process for preparing sulfonated arylphosphines by reaction of arylphosphines with oleum, wherein, after reaction of the arylphosphines with oleum, the sulfonation mixture is firstly diluted with water and cooled, an inert gas stream is then passed through the diluted sulfonation mixture until SO₂ is no longer liberated from the diluted sulfonation mixture and the latter is then worked up further in a customary manner.

2. The process as claimed in claim 1, wherein the starting compounds used for the sulfonation are arylphosphines such as monophosphines, diphosphines, oligophosphines and polyphosphines which contain at least one aromatic radical.

3. The process as claimed in claim 1 or 2, wherein the sulfonation mixture of arylphosphines and oleum is diluted with water to a sulfuric acid content of from 25 to 30%.

4. The process as claimed in any one of claims 1 to 3, wherein the diluted sulfonation mixture is cooled immediately and a continuous stream of inert gas is passed through the diluted sulfonation mixture.

5. The process as claimed in any one of claims 1 to 4, wherein inert gases used are nitrogen, the noble gases helium, neon, argon, xenon and krypton, carbon dioxide, carbon monoxide or hydrogen, preferably nitrogen or noble gases.

6. The process as claimed in any one of claims 1 to 5, wherein the amount of inert gas per kg of diluted sulfonation mixture is in the range from 5 to 500 l of inert gas, preferably from 10 to 200 l, particularly preferably from 20 to 100 l.

7. The process as claimed in any one of claims 1 to 6, wherein the duration of the gas treatment is in the range from 2 to 100 minutes, preferably from 2 to 20 minutes and where the sulfite content is < 0.1% by weight, preferably < 0.05% by weight, after customary work-up in the sulfonated arylphosphine solution.

8. The process as claimed in any one of claims 1 to 7, wherein the SO₂-laden inert gas stream is passed through a scrubbing solution in which the SO₂ is absorbed, with the scrubbing solution preferably being a basic liquid such as sodium carbonate solution or NaOH solution or waste sulfuric acid.

9. A process for preparing solutions of sulfonated arylphosphines obtained by reaction of arylphosphines with oleum and dilution of the sulfonation mixture with water, extraction of the diluted sulfonation mixture with the solution of a water-insoluble amine in a water-insoluble organic solvent, reaction of the organic phase with the solution of an inorganic base in water, wherein the resulting aqueous solution comprising sulfonated arylphosphines is brought to a pH of up to 3 by addition of an acid, and an inert gas stream is then passed through the aqueous solution comprising sulfonated arylphosphines until sulfur dioxide is no longer liberated from the aqueous solution comprising sulfonated arylphosphines.

10. The process as claimed in claim 9, wherein the arylphosphines used as starting compounds for the sulfonation are monophosphines, diphosphines, oligophosphines and polyphosphines which contain at least one aromatic radical.

11. The process as claimed in claim 9 or 10, wherein dimethylphenylphosphine, methyldiphenylphosphine and triphenylphosphine are used as monophosphines.

12. The process as claimed in any one of claims 9 to 11, wherein the aqueous solution comprising sulfonated arylphosphines is brought to a pH of 0-2 by addition of an acid.

13. The process as claimed in any one of claims 9 to 12, wherein the aqueous solution comprising sulfonated arylphosphines is admixed with inorganic or organic acids.

14. The process as claimed in any one of claims 9 to 13, wherein the acid used is sulfuric acid, phosphoric acid, nitric acid, hydrochloric acid, formic acid or acetic acid.

15. The process as claimed in any one of claims 9 to 14, wherein an inert gas stream is passed continuously or discontinuously through the aqueous solution comprising sulfonated arylphosphines.

16. The process as claimed in any one of claims 9 to 15, wherein inert gases used are nitrogen, the noble gases helium, neon, argon, xenon and krypton, carbon dioxide, carbon monoxide or hydrogen, preferably nitrogen or noble gases.

17. The process as claimed in any one of claims 9 to 16, wherein the continuous gas treatment is carried out for a time in the range from 2 to 100 minutes, preferably from 2 to 20 minutes, and the amount of inert gas per kg of aqueous solution comprising the sulfonated arylphosphine is in the range from 5 to 500 l of inert gas, preferably from 10 to 200 l, particularly preferably from 20 to 100 l of inert gas, the sulfite content in the aqueous solution comprising sulfonated arylphosphines being < 0.1% by weight, preferably < 0.05% by weight.

18. The process as claimed in any one of claims 9 to 17, wherein the discontinuous gas treatment is carried out for a time in the range from 1 to 100 hours, preferably from 5 to 50 hours and particularly preferably from 10 to 20 hours, and the amount of inert gas per kg of aqueous solution comprising the sulfonated arylphosphine is in the range from 5 to 1000 l of inert gas, preferably from 10 to 200 l, particularly preferably from 20 to 100 l of inert gas, the sulfite content in the aqueous solution comprising sulfonated arylphosphines being < 0.1% by weight, preferably < 0.05% by weight.

19. The process as claimed in any one of claims 9 to 18, wherein the inert gas stream laden with sulfur dioxide is passed through a scrubbing solution in which sulfur dioxide is absorbed, with the scrubbing solution preferably being a basic liquid such as sodium carbonate solution or sodium hydroxide solution or waste sulfuric acid.

20. An aqueous catalyst solution comprising rhodium and an aqueous solution comprising sulfonated aryl phosphines, obtainable as claimed in any of claims 9 to 19, for the hydroformylation of olefinically unsaturated compounds.

21. The use of an aqueous catalyst solution as claimed in claim 20 for the hydroformylation of olefinically unsaturated compounds.

## Revendications

1. Procédé de préparation d'arylphosphines sulfonées par réaction d'arylphosphines avec de l'oléum, **caractérisé en ce que**, après la réaction des arylphosphines avec l'oléum, on dilue d'abord le mélange de sulfonation avec de l'eau et on le refroidit, **en ce que** l'on fait ensuite passer un courant de gaz inerte dans le mélange de sulfonation dilué jusqu'à ce qu'il n'y ait plus de libération de SO₂ à partir du mélange de sulfonation dilué, et **en ce que** l'on continue ensuite le traitement de la manière habituelle.

2. Procédé selon la revendication 1, **caractérisé en ce que** l'on utilise comme composés de départ pour la sulfonation des arylphosphines comme des mono-, di-, oligo- et polyphosphines contenant au moins un reste aromatique.

3. Procédé selon la revendication 1 ou 2, **caractérisé en ce que** l'on effectue la dilution du mélange de sulfonation obtenu à partir d'arylphosphines et d'oléum avec de l'eau jusqu'à une teneur en acide sulfurique de 25 à 30 %.

4. Procédé selon l'une des revendications 1 à 3, **caractérisé en ce que** l'on refroidit aussitôt le mélange de sulfonation dilué et **en ce que** l'on fait passer un courant de gaz inerte continu dans le mélange de sulfonation dilué.

5. Procédé selon l'une des revendications 1 à 4, **caractérisé en ce que** l'on utilise comme gaz inerte de l'azote, les gaz rares hélium, néon, argon, xénon et krypton, du dioxyde de carbone, du monoxyde de carbone ou de l'hydrogène, de préférence de l'azote ou des gaz rares.

6. Procédé selon l'une des revendications 1 à 5, **caractérisé en ce que** l'on ajuste la quantité de gaz inerte dans un intervalle compris entre 5 et 500 L de gaz inerte, de préférence entre 10 et 200 L, de façon particulièrement préférée entre 20 et 100 L, barbotage par kg de mélange de sulfonation dilué.

7. Procédé selon l'une des revendications 1 à 6, **caractérisé en ce que** la durée du barbotage est comprise entre 2 et 100 minutes, de préférence entre 2 et 20 minutes, la teneur en sulfite de la solution d'arylphosphine sulfonée étant après le traitement habituel inférieure à 0,1 % en masse, de préférence inférieure à 0,05 % en masse.

8. Procédé selon l'une des revendications 1 à 7, **caractérisé en ce que** l'on fait passer le courant de gaz inerte chargé de SO₂ dans une solution de lavage absorbant le SO₂, la solution de lavage étant de préférence un liquide basique comme une solution de carbonate de sodium ou une solution de NaOH ou un acide sulfurique résiduaire.

9. Procédé de préparation de solutions d'arylphosphines sulfonées obtenues par réaction des arylphosphines avec de l'oléum et dilution du mélange de sulfonation avec de l'eau, extraction du mélange de sulfonation dilué avec une solution d'une amine insoluble dans l'eau dans un solvant organique insoluble dans l'eau, réaction de la phase organique avec une solution d'une base inorganique dans de l'eau, **caractérisé en ce que** l'on amène la solution aqueuse contenant les arylphosphines sulfonées obtenue à un pH allant jusqu'à 3 en ajoutant un acide, et **en ce que** l'on fait ensuite passer un courant de gaz inerte dans la solution aqueuse contenant les arylphosphines sulfonées jusqu'à ce qu'il n'y ait plus de libération de dioxyde de soufre à partir de la solution aqueuse contenant les arylphosphines sulfonées.

10. Procédé selon la revendication 9, **caractérisé en ce que** l'on utilise comme arylphosphines servant de composés de départ pour la sulfonation des mono-, di-, oligo- et polyphosphines contenant au moins un reste aromatique.

11. Procédé selon la revendication 9 ou 10, **caractérisé en ce que** l'on utilise comme monophosphines de la diméthylphénylphosphine, de la méthyldiphénylphosphine et de la triphénylphosphine.

12. Procédé selon l'une des revendications 9 à 11, **caractérisé en ce que** l'on amène la solution aqueuse contenant les arylphosphines sulfonées à un pH de 0 à 2 en ajoutant un acide.

13. Procédé selon l'une des revendications 9 à 12, **caractérisé en ce que** l'on ajoute un acide inorganique ou organique à la solution aqueuse contenant les arylphosphines sulfonées.

14. Procédé selon l'une des revendications 9 à 13, **caractérisé en ce que** l'on utilise comme acide l'acide sulfurique, l'acide phosphorique, l'acide nitrique, l'acide chlorhydrique, l'acide formique ou l'acide acétique.

15. Procédé selon l'une des revendications 9 à 14, **caractérisé en ce que** l'on fait passer un courant de gaz inerte en continu ou en discontinu dans la solution aqueuse contenant les arylphosphines sulfonées.

16. Procédé selon l'une des revendications 9 à 15, **caractérisé en ce que** l'on utilise comme gaz inerte de l'azote, les gaz rares hélium, néon, argon, xénon et krypton, du dioxyde de carbone, du monoxyde de carbone ou de l'hydrogène, de préférence de l'azote ou des gaz rares.

17. Procédé selon l'une des revendications 9 à 16, **caractérisé en ce que**, dans le cas du barbotage effectué en continu, la durée du barbotage est établie à une valeur comprise entre 2 et 100 minutes, de préférence entre 2 et 20 minutes, et la quantité de gaz inerte est établie à une valeur comprise entre 5 et 500 L de gaz inerte, de préférence entre 10 et 200 L, de façon particulièrement préférée entre 20 et 100 L de gaz inerte par kg de solution aqueuse contenant l'arylphosphine sulfonée, la teneur en sulfite de la solution aqueuse contenant les arylphosphines sulfonées étant inférieure à 0,1 % en masse, de préférence inférieure à 0,05 % en masse.

18. Procédé selon l'une des revendications 9 à 17, **caractérisé en ce que**, dans le cas d'un barbotage effectué en discontinu, la durée du barbotage est établie à une valeur comprise entre 1 et 100 heures, de préférence entre 5 et 50 heures et de façon particulièrement préférée entre 10 et 20 heures, et la quantité de gaz inerte est établie à une valeur comprise entre 5 et 1 000 L de gaz inerte, de préférence entre 10 et 200 L, de façon particulièrement préférée entre 20 et 100 L de gaz inerte par kg de solution aqueuse contenant l'arylphosphine sulfonée, la teneur en sulfite de la solution aqueuse contenant les arylphosphines sulfonées étant inférieure à 0,1 % en masse, de préférence inférieure à 0,05 % en masse.

19. Procédé selon l'une des revendications 9 à 18, **caractérisé en ce que** l'on fait passer le gaz inerte chargé de dioxyde de soufre dans une solution de lavage qui absorbe le dioxyde de soufre, la solution de lavage étant de préférence un liquide basique comme une solution de carbonate de sodium ou une solution d'hydroxyde de sodium ou un acide sulfurique résiduaire.

20. Solution aqueuse de catalyseur contenant du rhodium et une solution aqueuse contenant des arylphosphines sulfonées pouvant être obtenue selon l'une des revendications 9 à 19, pour l'hydroformylation de composés à insaturation oléfinique.

21. Utilisation de la solution aqueuse de catalyseur selon la revendication 20 pour l'hydroformylation de composés à insaturation oléfinique.
